# EUROPÄISCHE PATENTSCHRIFT

(11) **EP 1 819 315 B1**
(45) Veröffentlichungstag und Bekanntmachung des Hinweises auf die Patenterteilung: **28.01.2009**
(21) Anmeldenummer: 05749101.1
(22) Anmeldetag: 20.05.2005
(51) Int. Cl.: A61K 8/06, A61K 8/34, A61K 8/37, A61K 8/39, A61K 8/46, A61K 8/49, A61K 8/55, A61K 8/60, A61K 8/86, A61K 8/92, A61Q 17/04

(54) **2-PHENYLETHYLBENZOAT IN KOSMETISCHEN ÖL-IN-WASSER-UV-LICHTSCHUTZEMULSIONEN**
2-PHENYLETHYL BENZOATE IN COSMETIC OIL-IN-WATER UV-PROTECTION EMULSIONS
BENZOATE DE 2-PHENYLETHYLE DANS DES EMULSIONS COSMETIQUES DE PROTECTION CONTRE LES UV HUILE DANS EAU

(30) Priorität: 02.06.2004 DE 102004027476
(43) Veröffentlichungstag der Anmeldung: 22.08.2007
(73) Patentinhaber: Beiersdorf AG, 20253 Hamburg (DE)
(72) Erfinder: MUNDT, Claudia, 28207 Bremen (DE); CLAUSEN, Andreas, 20146 Hamburg (DE); HOOP, Kerstin, 25421 Pinneberg (DE); LERG, Heike, 22303 Hamburg (DE)
(86) Internationale Anmeldenummer: PCT/EP2005/052334
(87) Internationale Veröffentlichungsnummer: WO 2005/117824

(56) Entgegenhaltungen:
- WO-A-96/12467
- WO-A-03/039510
- WO-A-20/05009341
- DE-A1- 10 051 351

## Beschreibung

Die vorliegende Erfindung betrifft kosmetische eine Öl-in-Wasser-Lichtschutzemulsion enthaltend 2-Phenylethylbenzoat.

Der Trend weg von der vornehmen Blässe hin zur "gesunden, sportlich braunen Haut" ist seit Jahren ungebrochen. Um diese zu erzielen setzen die Menschen ihre Haut der Sonnenstrahlung aus, da diese eine Pigmentbildung im Sinne einer Melaninbildung hervorruft. Die ultraviolette Strahlung des Sonnenlichtes hat jedoch auch eine schädigende Wirkung auf die Haut. Neben der akuten Schädigung (Sonnenbrand) treten Langzeitschäden wie ein erhöhtes Risiko an Hautkrebs zu erkranken bei übermäßiger Bestrahlung mit Licht aus dem UVB-Bereich (Wellenlänge: 280-320 nm) auf. Die übermäßige Einwirkung der UVB-und UVA-Strahlung (Wellenlänge: 320-400 nm) führt darüber hinaus zu einer Schwächung der elastischen und kollagenen Fasern des Bindegewebes. Dies führt zu zahlreichen phototoxischen und photoallergischen Reaktionen und hat eine vorzeitige Hautalterung zur Folge.

Zum Schutz der Haut wurden daher eine Reihe von Lichtschutzfiltersubstanzen entwickelt, die in kosmetischen Zubereitungen eingesetzt werden können. Diese UVA- und UVB-Filter sind in den meisten Industrieländern in Form von Positivlisten wie dem Anlage 7 der Kosmetikverordnung zusammengefasst.

Eine besonders häufig werden kosmetische Lichtschutzzubereitungen in Form von Öl-in-Wasser-Emulsionen (O/W-Emulsionen) eingesetzt. Bei diesem Emulsionstyp liegen die Öltröpfchen fein verteilt in Wasser vor. Der Grundcharakter einer O/W-Emulsion ist durch das Wasser geprägt. Aufgrund ihres wässrigen Charakters ziehen O/W-Emulsionen besonders leicht in die Haut ein ohne einen lang anhaltenden, sensorisch unattraktiven, fettig-klebrigen Film auf der Haut hinterlassen.

Ein Nachteil solcher Lichtschutzzubereitungen auf O/W-Emulsionsbasis besteht jedoch darin, dass sich nur begrenzte Mengen an öllöslichen UV-Filtersubstanzen aus der Gruppe der Triazinderivate in die Zubereitung einarbeiten lassen, wodurch der Lichtschutzfaktor dieser Emulsionen begrenzt wird, wenn sich die Zubereitung nicht allzu fettig anfühlen soll. Auch kann es bei dem Einsatz größerer Mengen an Triazinderivaten zu Unverträglichkeiten mit dem Emulgatorsystem kommen.

Wegen seiner hervorragenden sensorischen Eigenschaften sowie seiner Spreitwirkung auf die Ölphase, werden in kosmetischen O/W-Emulsionen regelmäßig große Mengen an Cyclomethiconen eingesetzt. In cyclomethiconhaltige Ölphasen lassen sich jedoch nur begrenzte Mengen an Triazinderivaten einarbeiten.

Ein weiterer Nachteil an Zubereitungen auf O/W-Emulsionsbasis besteht darin, dass O/W-Emulsionen mit Konservierungsmitteln vor mikrobieller Zersetzung geschützt werden müssen. Obwohl die in der Kosmetik zugelassenen Konservierungsmittel aus medizinischer Sicht vollkommen unproblematisch anzusehen ist, können sie, wie letztlich jede chemische Substanz, in Einzelfällen zu Unverträglichkeiten führen. Generell besteht der Wunsch der Verbraucher nach möglichst konservierungsmittelarmen kosmetische Zubereitungen.

Nachteilig am Stande der Technik ist nicht zuletzt der Umstand, dass Lichtschutzemulsionen auf O/W-Basis allenfalls eine sehr begrenzte Wasserfestigkeit aufweisen. Sonnenschutzprodukte werden jedoch meist am Strand bzw. im Schwimmbad angewendet, und sollten idealer Weise auch nach dem Baden die Haut vor UV-Strahlung schützen.

Es war daher die Aufgabe der vorliegenden Erfindung die Nachteile des Standes der Technik zu beseitigen und kosmetische O/W-Lichtschulzemulsionen zu entwickeln,
- in die große Mengen an UV-Filtersubstanzen aus der Gruppe der Triazinderivate einarbeiten lassen,
- die sich aufgrund ihres Spreitvermögens gut auf der Haut verteilen lassen,
- die eine angenehme Sensorik aufweisen, sich nicht fettig oder klebrig anfühlen,
- der Haut an seidig mattes Aussehen verleihen,
- die eine erhöhte Wasserfestigkeit aufweisen,
- die mit einer im Vergleich zum Stand der Technik reduzierten Menge an Konservierungsmitteln vor der mikrobiellen Zersetzung geschützt werden können.

Ferner war es die Aufgabe der vorliegenden Erfindung kosmetische O/W-Lichtschutzemulsionen mit einem reduzierten Gehalt an Cyclomethiconen zu entwickeln bzw. Cyclomethicone in den Zubereitungen weitgehend zu ersetzen ohne das es zu Einbußen bei der sensorischen Performance der Zubereitung kommt.

Überraschend gelöst werden die Aufgaben durch eine kosmetische Öl-in-Wasser-Emulsion enthaltend
a) 2-Phenylethylbenzoat,
b) ein oder mehrere UV-Filtersubstanzen aus der Gruppe der Triazinderivate,
c) einen O/W-Emulgator oder eine O/W-Emulgatorkombination gewählt aus den folgenden O/W-Emulgatoren:
   i) Glycerylstearatcitrat,
   ii) Cetearylalkohol + Per-40 hydriertes Rizinusöl + Natriumcetearylsulfat + Glycerylstearat,
   iii) Polyglyceryl-3-methylglucosedistearat,
   iv) Stearinsäure
   v) PEG-40 Stearat
   vi) PEG-100 Stearat oder
   vii) Kaliumcetylphosphat.

Dabei ist es erfindungsgemäß bevorzugt, die O/W-Emulgatoren aus der Liste der folgenden Verbindungen zu wählen:
i) Glycerylstearatcitrat,
ii) Cetearytalkohol + PEG-40 hydriertes Rizinusöl + Natriumcetearylsulfat + Glycerylstearat,
iii) Polyglyceryl-3-methylglucosedistearat oder
iv) Stearinsäure
v) PEG-40 Stearat.

Erfindungsgemäß besonders bevorzugt ist der Einsatz der O/W-Emulgatoren Glycerylstearatcitrate+ PEG-40 hydriertes Rizinusöl + Natriumcetearylsulfat + Glycerylstearat, Polyglyceryl-3-methylglucosedistearat.

Die erfindungsgemäße Zubereitung bzw. erfindungsgemäße Verwendungen sind erfindungsgemäß vorteilhaft dadurch gekennzeichnet, dass sie 2-Phenylethylbenzoat in einer Konzentration von 0,5 bis 20 Gewichts-% und erfindungsgemäß bevorzugt von 2 bis 10 Gewichts-%, jeweils bezogen auf das Gesamtgewicht der Zubereitung, enthält.

Das erfindungsgemäße 2-Phenylethylbenzoat (INCl: 2-phenylethyl benzoate) weist die folgende Struktur auf 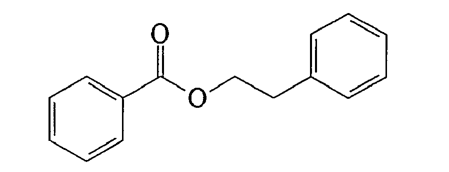 und ist beispielsweise unter dem Handelsnamen X-Tend™ 226 bei der Firma ISP erhältlich.

Die erfindungsgemäße Zubereitung bzw. erfindungsgemäße Verwendungen sind erfindungsgemäß vorteilhaft dadurch gekennzeichnet, dass die Zubereitung einen oder mehrere UV-Filtersubstanzen aus der Gruppe der Triazinderivate gewählt aus der Gruppe der Verbindungen
• 2,4-Bis-{[4-(2-ethyl-hexyloxy)-2-hydroxy]-phenyl}-6-(4-methoxyphenyl)-1,3,5-triazin (INCl: Aniso Triazin, erhältlich unter dem Handelsnamen Tinosorb S),
• Dioctylbutylamidotriazon (INCl: Dioctylbutamidotriazone),
• 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0, erhältlich bei 3V Sigma unter der Handelsbezeichnung Uvasorb® K2A),
• 4,4',4"-(1,3,5-Triazin-2,4,6-triyltriimino)-tris-benzoësäure-tris(2-ethylhexylester) (auch: 2,4,6-Tris-[anilino-(p-carbo-2'-ethyl-1'-hexyloxy)]-1,3,5-triazin (INCl: Octyl Triazone), welches von der BASF Aktiengesellschaft unter der Warenbezeichnung UVINUL® T 150 vertrieben wird).

Zu den erfindungsgemäß vorteilhaften Triazinderivaten gehören auch Bis-Resorcinyltriazinderivate. Sie zeichnen sich durch die folgende Strukturformel aus: 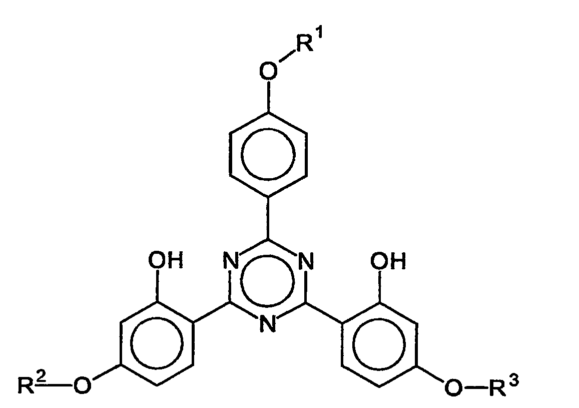 wobei R¹, R² und R³ unabhängig voneinander gewählt werden aus der Gruppe der verzweigten und unverzweigten Alkylgruppen mit 1 bis 10 Kohlenstoffatomen bzw. ein einzelnes Wasserstoffatom darstellen. Erfindungsgemäß besonders bevorzugt ist das 2,4-Bis-{[4-(2-Ethyl-hexyloxy)-2-hydroxy]-phenyl}-6-(4-methoxyphenyl)-1,3,5-triazin (INCl: Bis-Ethylhexyloxylphenol Methoxyphenyl Triazin), welches unter der Handelsbezeichnung Tinosorb® S bei der CIBA-Chemikalien GmbH erhältlich ist. Ein weiteres bevorzugtes Bis-Resorcinyltriazinderivat ist das 2-[4,6-Bis(2,4-dimethylphenyl)-1,3,5-triazin-2-yl]-5-(octyloxy)phenol (CAS Nr.: 2725-22-6).

Die Gesamtmenge an einem oder mehreren Bis-Resorcinyltriazinderivaten in den fertigen kosmetischen Zubereitungen wird vorteilhaft aus dem Bereich 0,01 Gew.% bis 20 Gew.-%, vorzugsweise von 0,1 bis 10 Gew.% gewählt, jeweils bezogen auf das Gesamtgewicht der Zubereitungen.

Erfindungsgemäß bevorzugt ist dabei der Einsatz von 2,4-Bis-{[4-(2-Ethyl-hexyloxy)-2-hydroxy]-phenyl}-6-(4-methoxyphenyl)-1,3,5-triazin als UV-Filtersubstanz aus der Gruppe der Triazinderivate.

Erfindungsgemäß vorteilhafte Ausführungsformen der vorliegenden Erfindung sind dadurch gekennzeichnet, dass sie UV-Filtersubstanzen aus der Gruppe der Triazinderivate in einer Konzentration von 0,01 bis 20 Gewichts-%, bezogen auf das Gesamtgewicht der Zubereitung, enthält. Dabei ist ein Gehalt an UV-Filtersubstanzen aus der Gruppe der Triazinderivate in einer Konzentration von 0,5 bis 10 Gewichts-%, bezogen auf das Gesamtgewicht der Zubereitung, erfindungsgemäß bevorzugt.

Es ist erfindungsgemäß vorteilhaft, wenn die Zubereitung oder Verwendung gemäß der vorliegenden Erfindung, weitere UV-Filtersubstanzen enthält.

Vorteilhafte weitere UV-Filtersubstanzen im Sinne der vorliegenden Erfindung sind die im folgenden genannten, welche in der Wasser- und/oder der Ölphase vorliegen können.

Vorteilhafte bei Raumtemperatur flüssige UV-Filtersubstanzen im Sinne der vorliegenden Erfindung sind, 2-Ethylhexyl-2-cyano-3,3-diphenylacrylat (INCl: Octocrylene) und Ester der Zimtsäure, vorzugsweise 4-Methoxyzimtsäure(2-ethylhexyl)ester (2-Ethylhexyl-4-methoxycinnamat, INCl: Octyl Methoxycinnamate) und 4-Methoxyzimtsäureisopentylester (Isopentyl-4-methoxycinnamat, INCl: Isoamyl p-Methoxycinnamate), 3-(4-(2,2-bis Ethoxycarbonylvinyl)-phenoxy)propenyl)-methoxysiloxan / Dimethytsiloxan - Copolymer welches beispielsweise unter der Handelsbezeichnung Parsol® SLX bei Hoffmann La Roche erhältlich ist.

Vorteilhafte UV-A-Filtersubstanzen im Sinne der vorliegenden Erfindung sind Dibenzoylmethanderivate, insbesondere das 4-(tert.-Butyl)-4'-methoxydibenzoylmethan (CAS-Nr. 70356-09-1), welches von Givaudan unter der Marke Parsol^{®} 1789 und von Merck unter der Handelsbezeichnung Eusolex® 9020 verkauft wird.

Weitere vorteilhafte UV-A-Filtersubstanzen im Sinne der vorliegenden Erfindung sind Hydroxybenzophenone, welche sich durch die folgende Strukturformel auszeichnen: 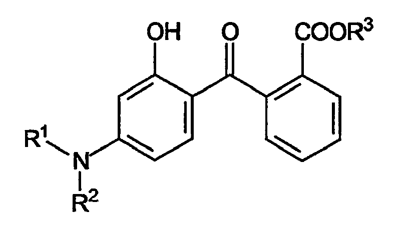 worin
R¹ und R² unabhängig voneinander Wasserstoff, C₁-C₂₀-Alkyl, C₃-C₁₀-Cycloalkyl oder C₃-C₁₀. Cycloalkenyl bedeuten, wobei die Substituenten R¹ und R² gemeinsam mit dem Stickstoffatom, an das sie gebunden sind, einen 5- oder 6-Ring bilden können und
R³ einen C₁-C₂₀-Alkyl Rest bedeutet.

Ein besonders vorteilhaftes Hydroxybenzophenon im Sinne der vorliegende Erfindung ist das 2-(4'-Diethylamino-2'-hydoxybenzoyl)-benzoesäurehexylester (auch: Aminobenzophenon), welches sich durch folgende Struktur auszeichnet: 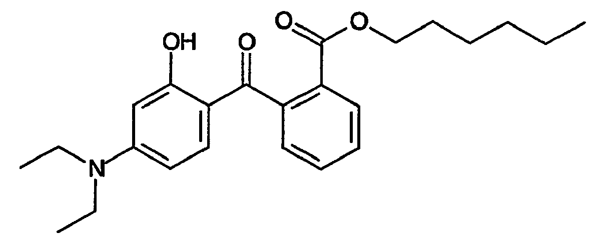 und unter dem Uvinul A Plus bei der Fa. BASF erhältlich ist.

Vorteilhafte weitere UV-Filtersubstanzen im Sinne der vorliegenden Erfindung sind sulfonierte, wasserlösliche UV-Filter, wie z. B.:
• Phenylen-1,4-bis-(2-benzimidazyl)-3,3'-5,5'-tetrasulfonsäure und ihre Salze, besonders die entsprechenden Natrium-, Kalium- oder Triethanolammonium-Salze, insbesondere das Phenylen-1,4-bis-(2-benzimidazyl)-3,3'-5,5'-tebasulfonsäure-bis-natriumsalz mit der INCl-Bezeichnung Bisimidazylate (CAS-Nr.: 180898-37-7), welches beispielsweise unter der Handelsbezeichnung Neo Heliopan AP bei Haarmann & Reimer erhältlich ist;
• Salze der 2-Phenylbenzimidazol-5-sulfonsäure, wie ihr Natrium-, Kalium- oder ihr Triethanolammonium-Salz sowie die Sulfonsäure selbst mit der INCl Bezeichnung Phenylbenzimidazole Sulfonsäure (CAS.-Nr. 27503-81-7), welches beispielsweise unter der Handelsbezeichnung Eusolex 232 bei Merck oder unter Neo Heliopan Hydro bei Haarmann & Reimer erhältlich ist;
• 1,4-di(2-oxo-10-Sulfo-3-bornylidenmethyl)-Benzol (auch: 3,3'-(1,4-Phenylendimethylene)-bis-(7,7-dimethyl-2-oxo-bicyclo-[2.2.1]hept-1-ylmethan Sulfonsäure) und dessen Salze (besonders die entprechenden 10-Sulfato-verbindungen, insbesondere das entsprechende Natrium-, Kalium- oder Triethanolammonium-Salz), das auch als Benzol-1,4-di(2-oxo-3-bomylidenmethyl-10-sulfonsäure) bezeichnet wird. Benzol-1,4-di(2-oxo-3-bomylidenmethyl-10-sulfonsäure) hat die INCl-Bezeichnung Terephtalidene Dicampher Sulfonsäure (CAS.-Nr.: 90457-82-2) und ist beispielsweise unter dem Handelsnamen Mexoryl SX von der Fa. Chimex erhältlich;
• Sulfonsäure-Derivate des 3-Benzylidencamphers, wie z. B. 4-(2-Oxo-3-bomylidenmethyl)-benzolsulfonsäure, 2-Methyl-5-(2-oxo-3-bornylidenmethyl)sulfonsäure und deren Salze.

Vorteilhafte UV-Filtersubstanzen im Sinne der vorliegenden Erfindung sind ferner sogenannte Breitbandfilter, d.h. Filtersubstanzen, die sowohl UV-A- als auch UV-B-Strahlung absorbieren.

Ein vorteilhafter Breitbandfilter im Sinne der vorliegenden Erfindung ist auch das 2,2'-Methylen-bis-(6-(2H-benzotriazol-2-yl)-4-(1,1,3,3-tetramethylbutyl)-phenol), welches unter der Handelsbezeichnung Tinosorb® M bei der CIBA-Chemikalien GmbH erhältlich ist.

Vorteilhafter Breitbandfilter im Sinne der vorliegenden Erfindung ist ferner das 2-(2H-benzotriazol-2-yl)-4-methyl-6-[2-methyl-3-[1,3,3,3-tetramethyl-1-[(trimethylsilyl)oxy]disiloxanyl]propyl]-phenol (CAS-Nr.: 155633-54-8) mit der INCl-Bezeichnung Drometrizole Trisiloxane.

Die weiteren UV-Filtersubstanzen können öllöslich oder wasserlöslich sein. Vorteilhafte öllösliche Filtersubstanzen sind z. B::
• 3-Benzylidencampher-Derivate, vorzugsweise 3-(4-Methylbenzyliden)campher, 3-Benzylidencampher;
• 4-Aminobenzoesäure-Derivate, vorzugsweise 4-(Dimethylamino)-benzoesäure(2-ethylhexyl)ester, 4-(Dimethylamino)benzoesäureamylester,
• Ester der Benzalmalonsäure, vorzugsweise 4-Methoxybenzalmalonsäuredi(2-ethylhexyl)-ester;
• Dioctylbutylamidotriazon (INCl: Diethylhexyl Butamido Triazone), welches unter der Handelsbezeichnung UVASORB HEB bei Sigma 3V erhältlich ist;
• Ester der Zimtsäure, vorzugsweise 4-Methoxyzimtsäure(2-ethylhexyl)ester, 4-Methoxy zimtsäureisopentylester;
• Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon sowie
• an Polymere gebundene UV-Filter.

Diese zusätzlichen UV-Filtersubstanzen können erfindungsgemäß vorteilhaft in einer Gesamtkonzentrationn von 0,01 bis 20 Gewichts-% und erfindungsgemäß bevorzugt in einer Gesamtkonzentration von 0,5 bis 10 Gewichts-%, jeweils bezogen auf das Gesamtgewicht er Zubereitung in dieser enthalten sein.

Ferner kann die erfindungsgemäße Zubereitung vorteilhaft UV-Lichtschutzfiltersubstanzen auf der Basis anorganischer Pigmente enthalten. Bevorzugte anorganische Pigmente sind Metalloxide und/oder andere in Wasser schwerlösliche oder unlösliche Metallverbindungen, insbesondere Oxide des Titans (TiO₂), Zinks (ZnO), Eisens (z. B. Fe₂O₃), Zirkoniums (ZrO₂), Siliciums (SiO₂), Mangans (z. B. MnO), Aluminiums (Al₂O₃), Cers (z. B. Ce₂O₃ Mischoxide der entsprechenden Metalle sowie Abmischungen aus solchen Oxiden sowie das Sulfat des Bariums (BaSO₄).

Die Pigmente können vorteilhaft im Sinne der vorliegenden Erfindung auch in Form kommerziell erhältlicher öliger oder wäßriger Vordispersionen zur Anwendung kommen. Diesen Vordispersionen können vorteilhaft Dispergierhilfsmittel und/oder Solubilisationsvermittler zugesetzt sein.

Die Pigmente können erfindungsgemäß vorteilhaft oberflächlich behandelt ("gecoatet") sein, wobei beispielsweise ein hydrophiler, amphiphiler oder hydrophober Charakter gebildet werden bzw. erhalten bleiben soll. Diese Oberflächenbehandlung kann darin bestehen, dass die Pigmente nach an sich bekannten Verfahren mit einer dünnen hydrophilen und/oder hydrophoben anorganischen und/oder organischen Schicht versehen werden. Die verschiedenen Oberflächenbeschichtungen können im Sinne der vorliegenden Erfindung auch Wasser enthalten.

Anorganische Oberflächenbeschichtungen im Sinne der vorliegenden Erfindung können bestehen aus Aluminiumoxid (Al₂O₃), Aluminiumhydroxid Al(OH)₃, bzw. Aluminiumoxidhydrat (auch: Alumina, CAS-Nr.: 1333-84-2), Natriumhexametaphosphat (NaPO₃)₆, Natriummetaphosphat (NaPO₃)ₙ, Siliciumdioxid (SiO₂) (auch: Silica, CAS-Nr.: 7631-86-9), Bariumsulfat (BaSO₄) oder Eisenoxid (Fe₂O₃). Diese anorganischen Oberflächenbeschichtungen können allein, in Kombination und/oder in Kombination mit organischen Beschichtungsmaterialien vorkommen.

Organische Oberflächenbeschichtungen im Sinne der vorliegenden Erfindung können bestehen aus pflanzlichem oder tierischem Aluminiumstearat, pflanzlicher oder tierischer Stearinsäure, Laurinsäure, Dimethylpolysiloxan (auch: Dimethicone), Methylpolysiloxan (Methicone), Simethicone (einem Gemisch aus Dimethylpolysiloxan mit einer durchschnittlichen Kettenlänge von 200 bis 350 Dimethylsiloxan-Einheiten und Silicagel) oder Alginsäure. Diese organischen Oberflächenbeschichtungen können allein, in Kombination und/oder in Kombination mit anorganischen Beschichtungsmaterialien vorkommen.
Erfindungsgemäß geeignete Zinkoxidpartikel und Vordispersionen von Zinkoxidpartikeln sind unter folgenden Handelsbezeichnungen bei den aufgeführten Firmen erhältlich:

| **Handelsname** | **Coating** | **Hersteller** |
|---|---|---|
| Z- Cote HP1 | 2% Dimethicone | BASF |
| Z- Cote | / | BASF |
| ZnO NDM | 5% Dimethicone | H&R |
| MZ-505S | 5% Methicone | Tayca Corporation |

Geeignete Titandioxidpartikel und Vordispersionen von Titandiöxidpartikeln sind unter folgenden Handelsbezeichnungen bei den aufgeführten Firmen erhältlich:

| **Handelsname** | **Coating** | **Hersteller** |
|---|---|---|
| MT-100TV | Aluminiumhydroxid / Stearinsäure | Tayca Corporation |
| MT-100Z | Aluminiumhydroxid / Stearinsäure | Tayca Corporation |
| Eusolex T-2000 | Alumina / Simethicone | Merck KgaA |
| Titandioxid T805 (Uvinul TiO₂) | Octyltrimethylsilan | Degussa |

Dies Wasserphase der O/W-Emulsion der erfindungsgemäßen Zubereitung kann vorteilhaft übliche kosmetische Hilfsstoffe enthalten, wie beispielsweise Alkohole, insbesondere solche niedriger C-Zahl, vorzugsweise Ethanol und/oder Isopropanol, Diole oder Polyole niedriger C-Zahl sowie deren Ether, vorzugsweise Propylenglykol, Glycerin, Butylenglykol, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, Polymere, Schaumstabilisatoren, Elektrolyte sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z. B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole [von der Fa. Bf. Goodrich], beispielsweise Carbopole der Typen 980, 981, 1382, 2984, 5984, ETD 2020, ETD 2050, Ultrez 10, jeweils einzeln oder in Kombination.

Die erfindungsgemäßen Zubereitungen können ferner vorteilhaft auch Selbstbräunungssubstanzen enthalten, wie beispielsweise Dihydroxyacteon und/oder Melaninderivate in Konzentrationen von 1 Gew.% bis zu 10 Gew.%, bezogen auf das Gesamtgewicht der Zubereitung.

Ferner vorteilhaft können die erfindungsgemäßen Zubereitungen auch Repellentien zum Schutz vor Mücken, Zecken und Spinnen und dergleichen enthalten. Vorteilhaft sind z. B. N,N-Diethyl-3-methylbenzamid (Handelsbezeichnung: Meta-delphene, "DEET"), Dimethylphtalat (Handelsbezeichnung: Palatinol M, DMP), 1-Piperidincarbonsäure-2-(2-hydroxyethyl)-1-methylpropylester sowie insbesondere 3-(N-n-Butyl-N-acetyl-amino)-propionsäureethylester (unter dem Handelsnamen Insekt Repellent® 3535 bei der Fa. Merck erhältlich). Die Repellentien können sowohl einzeln als auch in Kombination eingesetzt werden.

Als Feuchthaltemittel (Moisturizer) werden Stoffe oder Stoffgemische bezeichnet, welche kosmetischen Zubereitungen die Eigenschaft verleihen, nach dem Auftragen bzw. Verteilen auf der Hautoberfläche die Feuchtigkeitsabgabe der Homschicht (auch transepidermal water loss (TEWL) genannt) zu reduzieren und/oder die Hydratation der Hornschicht positiv zu beeinflussen.

Vorteilhafte Feuchthaltemittel (Moisturizer) im Sinne der vorliegenden Erfindung sind beispielsweise Glycerin, Milchsäure und/oder Lactate, insbesondere Natriumlactat, Butylenglykol, Propylenglykol, Biosaccaride Gum-1, Glycine Soja, Ethylhexyloxyglycerin, Pyrrolidoncarbonsäure und Harnstoff. Ferner ist es insbesondere von Vorteil, polymere Moisturizer aus der Gruppe der wasserlöslichen und/oder in Wasser quellbaren und/oder mit Hilfe von Wasser gelierbaren Polysaccharide zu verwenden. Insbesondere vorteilhaft sind beispielsweise Hyaluronsäure, Chitosan und/oder ein fucosereiches Polysaccharid, welches in den Chemical Abstracts unter der Registraturnummer 178463-23-5 abgelegt und z. B. unter der Bezeichnung Fucogel®1000 von der Gesellschaft SOLABIA S.A. erhältlich ist. Moisturizer können vorteilhaft auch als Antifaltenwirkstoffe zum Schutz vor Hautveränderungen, wie sie z. B. bei der Hautalterung auftreten, verwendet werden.

Es ist vorteilhaft im Sinne der vorliegenden Erfindung, wenn die erfindungsgemäße Zubereitung ein oder mehrere Feuchthaltemittel in einer Gesamtkonzentration von 0,1 bis 20 Gewichts-% und bevorzugt in einer Gesamtkonzentration von 0,5 bis 10 Gewichts-%, jeweils bezogen auf das Gesamtgewicht der Zubereitung, enthält.

Die erfindungsgemäßen kosmetischen Zubereitungen können ferner vorteilhaft, wenngleich nicht zwingend, Füllstoffe enthalten, welche z. B. die sensorischen und kosmetischen Eigenschaften der Formulierungen weiter verbessern und beispielsweise ein samtiges oder seidiges Hautgefühl hervorrufen oder verstärken. Vorteilhafte Füllstoffe im Sinne der vorliegenden Erfindung sind Stärke und Stärkederivate (wie z. B. Tapiocastärke, Distärkephosphat, Aluminium- bzw. Natrium-Stärke Octenylsuccinat und dergleichen), Pigmente, die weder hauptsächlich UV-Filter- noch färbende Wirkung haben (wie z. B. Bornitrid etc.) und/oder Aerosile^{®} (CAS-Nr. 7631-86-9).

Die Ölphase der O/W-Emulsion der erfindungsgemäßen Zubereitung wird vorteilhaft gewählt aus der Gruppe der polaren Öle, beispielsweise aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z. B. Cocoglycerid, Olivenöl, Sonnenblumenöl, Sojaöl, Erdnußöl, Rapsöl, Mandelöl, Palmöl, Kokosöl, Rizinusöl, Weizenkeimöl, Traubenkernöl, Distelöl, Nachtkerzenöl, Macadamianußöl und dergleichen mehr.

Erfindungsgemäß vorteilhaft sind ferner z. B. natürliche Wachse tierischen und pflanzlichen Ursprungs, wie beispielsweise Bienenwachs und andere Insektenwachse sowie Beerenwachs, Sheabutter und/oder Lanolin (Wollwachs).

Weitere vorteilhafte polare Ölkomponenten können im Sinne der vorliegenden Erfindung ferner gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Octylpalmitat, Octylcocoat, Octylisostearat, Octyldodeceylmyristat, Octyldodekanol, Cetearylisononanoat, Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Stearylheptanoat, Oleyloleat, Oleylerucat, Erucyloleat, Erucylerucat, Tridecylstearat, Tridecyltrimellitat, sowie synthetische, halbsynthetische und natürliche Gemische solcher Ester, wie z. B. Jojobaöl.

Ferner kann die Ölphase vorteilhaft gewählt werden aus der Gruppe der Dialkylether und Dialkylcarbonate, vorteilhaft sind z. B. Dicaprylylether (*Cetiol OE*) und/oder Dicaprylylcarbonat, beispielsweise das unter der Handelsbezeichnung *Cetiol CC* bei der Fa. Cognis erhältliche.

Es ist ferner bevorzugt, das oder die Ölkomponenten aus der Gruppe Isoeikosan, Neopentylglykoldiheptanoat, Propylenglykoldicaprylat/dicaprat, Caprylic/Capric/Diglycerylsuccinat, Butylenglykol Dicaprylat/Dicaprat, C₁₂₋₁₃-Alkyllactat, Di-C₁₂₋₁₃-Alkyltartrat, Triisostearin, Dipentaerythrityl Hexacaprylat/Hexacaprat, Propylenglykolmonoisostearat, Tricaprylin, Dimethylisosorbid. Es ist insbesondere vorteilhaft, wenn die Ölphase der erfindungsgemäßen Formulierungen einen Gehalt an C₁₂₋₁₅-Alkylbenzoat aufweist oder vollständig aus diesem besteht.

Vorteilhafte Ölkomponenten sind ferner z. B. Butyloctylsalicylat (beispielsweise das unter der Handelsbezeichnung *Hallbrite BHB* bei der Fa. CP Hall erhältliche), Hexadecylbenzoat und Butyloctylbenzoat und Gemische davon (*Hallstar AB*) und/oder Diethylhexylnaphthalat (*Hallbrite TQ oder Corapan TQ von H&R*).

Auch beliebige Abmischungen solcher Öl- und Wachskomponenten sind vorteilhaft im Sinne der vorliegenden Erfindung einzusetzen.

Ferner kann die Ölphase ebenfalls vorteilhaft auch unpolare Öle enthalten, beispielsweise solche, welche gewählt werden aus der Gruppe der verzweigten und unverzweigten Kohlenwasserstoffe und -wachse, insbesondere Mineralöl, Vaseline (Petrolatum), Paraffinöl, Squalan und Squalen, Polyolefine, hydrogenierte Polyisobutene und Isohexadecan. Unter den Polyolefinen sind Polydecene die bevorzugten Substanzen.

Vorteilhaft kann die Ölphase ferner einen Gehalt an zyklischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Silikonöle sind hochmolekulare synthetische polymere Verbindungen, in denen SiliciumAtome über Sauerstoff-Atome ketten- und/oder netzartig verknüpft und die restlichen Valenzen des Siliciums durch Kohlenwasserstoff-Reste (meist Methyl-, seltener Ethyl-, Propyl-, Phenyl-Gruppen u. a.) abgesättigt sind. Systematisch werden die Silikonöle als Polyorganosiloxane bezeichnet. Die methylsubstituierten Polyorganosiloxane, welche die mengenmäßig bedeutendsten Verbindungen dieser Gruppe darstellen und sich durch die folgende Strukturformel auszeichnen 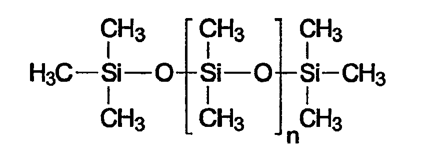 werden auch als Polydimethylsiloxan bzw. Dimethicon (INCl) bezeichnet. Dimethicone gibt es in verschiedenen Kettenlängen bzw. mit verschiedenen Molekulargewichten.

Besonders vorteilhafte Polyorganosiloxane im Sinne der vorliegenden Erfindung sind beispielsweise Dimethylpolysiloxane [Poly(dimethylsiloxan)], welche beispielsweise unter den Handelsbezeichnungen Abil 10 bis 10 000 bei Th. Goldschmidt erhältlich sind. Ferner vorteilhaft sind Phenylmethylpolysiloxane (INCl: Phenyl Dimethicone, Phenyl Trimethicone), zyklische Silikone (Octamethylcyclotetrasiloxan bzw. Decamethylcyclopentasiloxan), welche nach INCl auch als Cyclomethicone bezeichnet werden, aminomodifizierte Silikone (INCl: Amodimethicone) und Silikonwachse, z. B. Polysiloxan-Polyalkylen-Copolymere (INCl: Stearyl Dimethicone und Cetyl Dimethicone) und Dialkoxydimethylpolysiloxane (Stearoxy Dimethicone und Behenoxy Stearyl Dimethicone), welche als verschiedene Abil-Wax-Typen bei Th. Goldschmidt erhältlich sind. Aber auch andere Silikonöle sind vorteilhaft im Sinne der vorliegenden Erfindung zu verwenden, beispielsweise Cetyldimethicon, Hexamethylcyclotrisiloxan, Polydimethylsiloxan, Poly(methylphenylsiloxan).

Die erfindungsgemäßen Zubereitungen können ferner vorteilhaft eine oder mehrere Substanzen aus der folgenden Gruppe der Siloxanelastomere enthalten, beispielsweise um die Wasserfestigkeit und/oder den Lichtschutzfaktor der Produkte zu steigern:
(a) Siloxanelastomere, welche die Einheiten R₂SiO und RSiO_{1,5} und/oder R₃SiO_{0,5} und/oder SiO₂ enthalten,
   wobei die einzelnen Reste R jeweils unabhängig voneinander Wasserstoff, C₁₋₂₄-Alkyl (wie beispielsweise Methyl, Ethyl, Propyl) oder Aryl (wie beispielsweise Phenyl oder Tolyl), Alkenyl (wie beispielsweise Vinyl) bedeuten und das Gewichtsverhältnis der Einheiten R₂SiO zu RSiO_{1,5} aus dem Bereich von 1 : 1 bis 30 : 1 gewählt wird;
(b) Siloxanelastomere, welche in Silikonöl unlöslich und quellfähig sind, die durch die Additionsreaktion eines Organopolysiloxans (1), das siliciumbebundenen Wasserstoff enthält, mit einem Organopolysiloxan (2), das ungesättigte aliphatische Gruppen enthält, erhältlich sind,
wobei die verwendeten Mengenateile so gewählt werden, dass die Menge des Wasserstoffes des Organopolysiloxans (1) oder der ungesättigten aliphatischen Gruppen des Organopolysiloxans (2)
• im Bereich von 1 bis 20 mol-% liegt, wenn das Organopolysiloxan nicht zyklisch ist und
• im Bereich von 1 bis 50 mol-% liegt, wenn das Organopolysiloxan zyklisch ist

Vorteilhaft im Sinne der vorliegenden Erfindung liegen das oder die Siloxanelastomere in Form sphärischer Puder oder in Form von Gelen vor.

Erfindungsgemäß vorteilhafte in Form sphärischer Puder vorliegende Siloxanelastomere sind die mit der INCl-Bezeichnung Dimethicone / Vinyl Dimethicone Crosspolymer, beispielsweise das von DOW CORNING unter der Handelsbezeichnungen DOW CORNING 9506 Powder erhältliche.

Besonders bevorzugt ist es, wenn das Siloxanelastomer in Kombination mit Ölen aus Kohlenwasserstoffen tierischer und/oder pflanzlicher Herkunft, synthetischen Ölen, synthetischen Estern, synthetischen Ethern oder deren Gemischen verwendet wird.

Ganz besonders bevorzugt ist es, wenn das Siloxanelastomer in Kombination mit unverzweigten bei Raumtemperatur flüssigen oder pastösen Silikonölen oder zyklischen Silikonölen oder deren Gemischen verwendet wird. Insbesondere vorteilhaft sind Organopolysiloxanelastomere mit der INCl-Bezeichnung Dimethicone / Polysilicone-11, ganz besonders die von der Grant Industries Inc. erhältlichen Gransil-Typen GCM, GCM-5, DMG-6, CSE Gel, PM-Gel, LTX, ININ Gel, AM-18 Gel und/oder DMCM-5.

Ganz außergewöhnlich bevorzugt ist es, wenn das Siloxanelastomer in Form eines Gels aus Siloxanelastomer und einer Lipidphase verwendet wird, wobei der Gehalt des Siloxanelastomers in dem Gel 1 bis 80 Gew.%, bevorzugt 0,1 bis 60 Gew.% beträgt, jeweils bezogen auf das Gesamtgewicht des Gels.

Es ist vorteilhaft im Sinne der vorliegenden Erfindung, die Gesamtmenge der Siloxanelastomere (Aktivgehalt) aus dem Bereich von 0,01 bis 10 Gew.%, vorteilhaft von 0,1 bis 5 Gew.-%, jeweils bezogen auf das Gesamtgewicht der Formulierung, zu wählen.

Besonders vorteilhafte Zubereitungen werden ferner erhalten, wenn als Zusatz- oder Wirkstoffe Antioxidantien eingesetzt werden. Erfindungsgemäß enthalten die Zubereitungen vorteilhaft eines oder mehrere Antioxidantien. Als günstige, aber dennoch fakultativ zu verwendende Antioxidantien können alle für kosmetische Anwendungen geeigneten oder gebräuchlichen Antioxidantien verwendet werden.

Besonders vorteilhaft im Sinne der vorliegenden Erfindung können wasserlösliche Antioxidantien eingesetzt werden, wie beispielsweise Vitamine, z. B. Ascorbinsäure und deren Derivate.

Bevorzugte Antioxidantien sind ferner Vitamin E und dessen Derivate sowie Vitamin A und dessen Derivate.

Die Menge der Antioxidantien (eine oder mehrere Verbindungen) in den Zubereitungen beträgt vorzugsweise 0,001 bis 30 Gew.%, besonders bevorzugt 0,05 bis 20 Gew.%, insbesondere 0,1 bis 10 Gew.%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 bis 10 Gew.%, bezogen auf das Gesamtgewicht der Formulierung, zu wählen.

Sofern Vitamin A bzw. Vitamin-A-Derivate, bzw. Carotine bzw. deren Derivate das oder die Antioxidantien darstellen, ist vorteilhaft, deren jeweilige Konzentrationen aus dem Bereich von 0,001 bis 10 Gew.%, bezogen auf das Gesamtgewicht der Formulierung, zu wählen.

Es ist insbesondere vorteilhaft, wenn die kosmetischen Zubereitungen gemäß der vorliegenden Erfindung kosmetische Wirkstoffe enthalten, wobei bevorzugte Wirkstoffe Antioxidantien sind, welche die Haut vor oxidativer Beanspruchung schützen können.

Weitere vorteilhafte Wirkstoffe im Sinne der vorliegenden Erfindung sind natürliche Wirkstoffe und/oder deren Derivate, wie z. B. alpha-Liponsäure, Phytoen, D-Biotin, Coenzym Q10, alpha-Glucosylrutin, Carnitin, Carnosin, natürliche und/oder synthetische Isoflavonoide, Kreatin, Kreatinin, Taurin und/oder β-Alanin sowie 8-Hexadecen-1,16-dicarbonsäure (Dioic acid, CAS-Nummer 20701-68-2; vorläufige INCl-Bezeichnung Octadecendioic acid) und/oder Licochalcon A, welches sich durch die folgende Strukturformel auszeichnet:

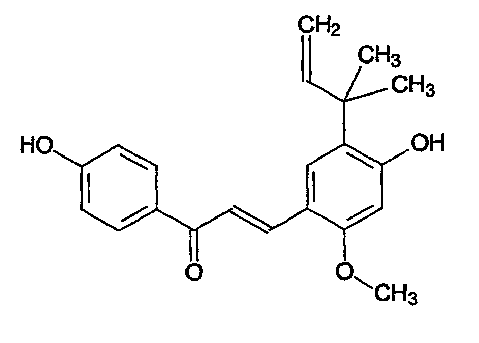

Licochalcon kann vorteilhaft auch als Bestandteil von pflanzlichen Extrakten, insbesondere von wäßrigen *Radix Glycyrrhizae inflatae,* eingesetzt werden.

Es ist erfindungsgemäß vorteilhaft, wenn die kosmetischen Zubereitungen 0,001 bis 10 Gew.-%, insbesondere 0,05 bis 5 Gew.%, ganz besonders 0,01 bis 2 Gew.% an einem Extrakt aus *Radix Glycyrrhizae inflatae* enthalten, jeweils bezogen auf das Gesamtgewicht der Zubereitung.

Ganz besonders vorteilhaft ist es, von einem Extrakt auszugehen, der unter der Bezeichnung Polyol Soluble Licorice Extract PU (INCl-Bezeichnung Glycyrrhiza Inflata) von der Firma Maruzen zu erhalten ist. Der Extrakt aus *Radix Glycyrrhizae inflatae* enthält einen Anteil von ca. 25 % Licochalcone A.

Erfindungsgemäße Rezepturen, welche z. B. bekannte Antifaltenwirkstoffe wie Flavonglycoside (insbesondere α-Glycosylrutin), Coenzym Q10, Vitamin E und/oder Derivate und dergleichen enthalten, eignen sich insbesondere vorteilhaft zum Schutz vor ästhetisch unattraktiven Hautveränderungen, wie sie z. B. bei der Hautalterung auftreten (wie beispielsweise Trockenheit, Rauhigkeit und Ausbildung von Trockenheitsfältchen, Juckreiz, verminderte Rückfettung (z. B. nach dem Waschen), sichtbare Gefäßerweiterungen (Teleangiektasien, Cuperosis), Schlaffheit und Ausbildung von Falten und Fältchen, lokale Hyper-, Hypo- und Fehlpigmentierungen (z. B. Altersflecken), vergrößerte Anfälligkeit gegenüber mechanischem Stress (z. B. Rissigkeit) und dergleichen). Weiterhin vorteilhaft eignen sie sich gegen das Erscheinungsbild der trockenen bzw. rauhen Haut.

Die kosmetischen Zubereitungen gemäß der Erfindung können kosmetische Hilfsstoffe enthalten, wie sie üblicherweise in solchen Zubereitungen verwendet werden, z. B. Konservierungsmittel, Konservierungshelfer, Komplexbildner, Bakterizide, Parfüme, Substanzen zum Verhindern oder Steigern des Schäumens, Farbstoffe, Pigmente, die eine färbende Wirkung haben, Verdickungsmittel, anfeuchtende und/oder feuchhaltende Substanzen, Füllstoffe, die das Hautgefühl verbessern, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Erfindungsgemäß bevorzugt sind Zubereitungen, die frei sind von Konservierungsstoffen.

Vorteilhaft im Sinne der vorliegenden Erfindung sind Zubereitungen zur Pflege der Haut: sie können dem kosmetischen Lichtschutz, ferner als Schminkprodukt in der dekorativen Kosmetik dienen. Eine weitere vorteilhafte Ausführungsform der vorliegenden Erfindung besteht in After-Sun-Produkten.

Entsprechend ihrem Aufbau können kosmetische Zusammensetzungen im Sinne der vorliegenden Erfindung, beispielsweise verwendet werden als Hautschutzcrème, Tages- oder Nachtcrème usw. Es ist gegebenenfalls möglich und vorteilhaft, die erfindungsgemäßen Zusammensetzungen als Grundlage für pharmazeutische Formulierungen zu verwenden.

Es ist auch vorteilhaft im Sinne der vorliegenden Erfindung, kosmetische Zubereitungen zu erstellen, deren hauptsächlicher Zweck nicht der Schutz vor Sonnenlicht ist, die aber dennoch einen Gehalt an UV-Schutzsubstanzen enthalten. So werden z. B. in Tagescrèmes oder Makeup-Produkten gewöhnlich UV-A- bzw. UV-B-Filtersubstanzen eingearbeitet. Auch stellen UV-Schutzsubstanzen, ebenso wie Antioxidantien und, gewünschtenfalls, Konservierungsstoffe, einen wirksamen Schutz der Zubereitungen selbst gegen Verderb dar. Günstig sind ferner kosmetische Zubereitungen, die in der Form eines Sonnenschutzmittels vorliegen.

Zur Anwendung werden die erfindungsgemäßen kosmetischen Zubereitungen in der für Kosmetika üblichen Weise auf die Haut und/oder die Haare in ausreichender Menge aufgebracht.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

### Beispiele

In den folgenden Beispielen bedeutet:
• UVASorb® K2A = 2,4-bis-[5-1(dimethylpropyl)benzoxazol-2-yl-(4-phenyl)-imino]-6-(2-ethylhexyl)-imino-1,3,5-triazin [CAS Nr. 288254-16-0]
• Uvinul® A Plus = 2-(4'-Diethylamino-2'-hydoxybenzoyl)-benzoesäurehexylester (auch: Aminobenzophenon)

**O/W Emulsionen**

| | **1** | **2** | **3** | **4** | **5** | **6** | **7** |
|---|---|---|---|---|---|---|---|
| PEG-40 Castor Oil, Natrium Cetearyl Sulfat Cetearyl Alkohol | | | | | | | 2,50 |
| Glycerinmonostearat (SE) | 1,00 | 2,00 | 3,00 | 1,00 | | 1,50 | |
| Glyceryl Stearat Citrat | | | | | 2,00 | | |
| Stearinsäure | 3,00 | | 2,50 | 2,00 | | | |
| PEG-40 Stearat | | 2,00 | | | | 2,00 | |
| PEG-100 Stearat | | | 0,75 | | | | |
| Lauryl Methicon Copolyol | | | | 0,75 | | 0,50 | |
| Sorbitan Stearat | | | 0,75 | | | | |
| Cetyl Phosphat | | | 0,75 | | | | |
| Stearyl Alkohol | | | 3,00 | | 2,00 | 2,00 | 0,50 |
| Cetyl Alkohol | 1,00 | 2,00 | | | 0,50 | | 2,00 |
| UVASorb® K2A | | | | 4,00 | | 5,00 | |
| Uvinul® A Plus | 2,50 | | | 0,25 | 1,00 | 0,50 | |
| Butylmethoxydibenzoylmethan | | | | | | | 4,00 |
| 4-Methylbenzylidencampher | | | 3,00 | | | | |
| Bis-Ethylhexyloxyphenol Methoxyphenyl Triazin | 1,00 | | | | | 1,00 | 0,50 |
| Dinatrium Phenyl Dibenzimidazol Tetrasulfonat | | | | 1,00 | | 2,00 | |
| Phenylbenzmidazol Sulfonsäure | | | | | | | 3,00 |
| Ethylhexyl Triazon | | | | 2,00 | | 2,00 | |
| Diethylhexyl Butamido Triazon | 2,00 | | | | | | |
| Ethylhexyl Methoxycinnamat | 3,50 | | | 10,00 | | | |
| Octocrylen | | | | 5,00 | 9,00 | 7,50 | 2,50 |
| Methylen Bis-Benztriazolyl Tetramethylbutylphenol | | | 2,00 | | 3,00 | | |
| Ethylhexylsalicylat | | 0,50 | 3,00 | | | | 5,00 |
| Drometrizol Trisiloxan | | | 0,50 | | | 1,00 | |
| Terephtalidene Dicampher Sulfonsäure | | | | | | 2,00 | |
| Dimethylcodiethylbenzalmalona t | | | | | | 3,00 | |
| Titandioxid T 805 | 2,00 | | | | 1,00 | 0,50 | |
| Titandioxid MT-100Z | | | | 3,00 | 1,00 | | |
| C₁₂₋₁₅ Alkyl Benzoat | 1,00 | | | | | 2,00 | |
| 2-Phenylethyl Benzoat | 1,50 | | | 0,50 | | 4,00 | 10,00 |
| Dicaprylylether | | | 3,50 | | 2,00 | | |
| Paraffinöl | | 6,00 | | | | | |
| Butylenglycol Dicaprylat/ Dicaprat | | | | 5,00 | 3,00 | | |
| Cetearyl Isononanoat | 4,00 | | | | | 2,00 | 2,00 |
| Dimethicon | 0,50 | 3,00 | 1,00 | | 2,00 | | |
| Cyclomethicon | | 3,00 | | 4,50 | | | 0,50 |
| Dimethicon / Vinyl Dimethicon Crosspolymer | 4,00 | | | | | | 0,50 |
| PVP Hexadecen Copolymer | | | | 0,50 | 1,00 | | 1,00 |
| Glycerin | 7,50 | 10,00 | | 7,50 | 5,00 | | 20,00 |
| Xanthangummi | | 0,20 | 0,05 | | | | 0,30 |
| Butylenglycol | 5,00 | | | | | 7,00 | |
| Ascorbinsäure | | | | | | 3,50 | |
| Tocopherol | 0,20 | | | | | | |
| Taurin | 1,00 | | | | | | |
| Vitamin E Acetat | | 0,40 | 0,25 | 0,50 | 0,75 | | 1,00 |
| Retinol | | | | | 0,05 | | |
| Dioic Acid (Octadecendicarbonsäure) | | | | 0,20 | | 0,25 | |
| Pyridoxin | | 0,5 | | | | | |
| Fucogel® 1000 | | | 1,50 | | | 5,00 | |
| Dihydroxyaceton | | 5,00 | | | | | |
| DMDM Hydantoin | 0,60 | | 0,40 | 0,20 | | | |
| Methylparaben | | 0,10 | 0,25 | | 0,50 | | |
| Phenoxyethanol | 0,40 | 0,50 | | 0,40 | 0,50 | | 0,60 |
| EDTA | 0,20 | | 0,35 | 0,50 | 0,02 | | 0,03 |
| Ethanol | 2,00 | | 1,50 | | 3,00 | 5,00 | 1,00 |
| Insekt Repellent 3535 | | | 5,00 | | | | |
| Parfüm | 0,20 | 0,20 | | | | 0,30 | 0,40 |
| Wasser | ad 100 | ad 100 | ad 100 | ad 100 | ad 100 | ad 100 | ad 100 |
| Neutralisationsmittel (Natriumhydroxid, Kaliumhydroxid) | qs | qs | qs | qs | qs | qs | qs |
| pH-Wert | 6,0-7,5 | 4,5-7,0 | 6,5-8,5 | 5,0-7,0 | 6,0-8,0 | 4,0-6,0 | 5,0-7,5 |

**O/W Emulsionen**

| | **8** | **9** | **10** | **11** | **12** |
|---|---|---|---|---|---|
| Glycerinmonostearat (SE) | | | | 1,00 | |
| Glyceryl Stearat Citrat | 2,00 | 2,00 | 1,50 | | |
| Polyglyceryl-3- Methylglucose Distearat | | | | | 4,50 |
| Stearyl Alkohol | 2,00 | 2,00 | | | |
| Cetyl Alkohol | | | 2,00 | | 4,50 |
| Bis-Ethylhexyloxyphenol Methoxyphenyl Triazin | 2,00 | 2,00 | 1,50 | | |
| Diethylhexyl Butamido Triazon | | 1,00 | 2,00 | | |
| Ethylhexyl Methoxycinnamat | 2,00 | 6,00 | 5,00 | | |
| Octocrylen | 2,00 | 9,00 | | | |
| Titandioxid T 805 | | 3,00 | 2,00 | | |
| C₁₂₋₁₅ Alkyl Benzoat | | | 3,00 | 3,00 | 1,00 |
| 2-Phenylethyl Benzoat | 3,00 | | | 5,00 | 10,00 |
| Hydriertes Kokosfettsäureglycerid | 1,00 | 1,00 | 3,00 | | |
| Dicaprylylether | 5,00 | 2,00 | 6,00 | | |
| Octyldodecanol | 6,00 | 5,00 | 4,00 | 1,00 | 2,00 |
| Butylenglycol Dicaprylat/ Dicaprat | 2,00 | 2,00 | | | |
| Caprylsäure/Caprinsäuretriglyc erid | | | | 2,00 | 1,50 |
| Dimethicon | | | 2,00 | | |
| Cyclomethicon | 2,00 | 1,00 | | 3,00 | |
| Sorbitol | | | | 2,50 | |
| Acrylat/C10-30 Alkyl Acrylat Crosspolymer | 0,10 | 0,10 | 0,05 | | |
| PVP Hexadecen Copolymer | 0,50 | 0,50 | | | |
| Glycerin | 8,00 | 6,00 | 5,00 | 3,00 | 3,00 |
| Xanthangummi | 0,40 | 0,40 | 0,25 | 0,30 | 0,10 |
| Butylenglycol | | | 3,00 | 3,00 | |
| Linolsäure | 1,00 | | | | |
| Nachtkerzenöl | | 1,00 | | | |
| Genistein | 0,05 | | | | |
| Silymarin | | 0,1 | | | |
| Phosphatidylcholin | | | | 0,06 | |
| Silybin | | | | 0,03 | |
| Carnitin | | | 0,5 | | |
| Vitamin E Acetat | 0,50 | | 0,30 | 0,40 | 0,40 |
| Dihydroxyaceton | | | | 5,00 | 4,00 |
| DMDM Hydantoin | 0,60 | 0,60 | 0,50 | | |
| Methylparaben | | | | 0,30 | 0,30 |
| Phenoxyethanol | 0,40 | 0,40 | 0,35 | 0,50 | 0,50 |
| EDTA | 1,00 | 1,00 | 1,00 | | 1,00 |
| Ethanol | | | 3,00 | 3,00 | |
| Insekt Repellent 3535 | | | | 4,00 | 5,00 |
| Parfüm | 0,40 | 0,40 | 0,40 | 0,40 | 0,40 |
| Wasser | ad 100 | ad 100 | ad 100 | ad 100 | ad 100 |
| Neutralisationsmittel (Natriumhydroxid, Kaliumhydroxid) | qs | qs | qs | qs | qs |
| pH-Wert | 5,0-7,0 | 5,0-7,0 | 5,0-7,0 | 5,0-7,0 | 5,0-7,0 |

**O/W-Emulsionen**

| | **13** | **14** | **15** | **16** | **17** | **18** | **19** |
|---|---|---|---|---|---|---|---|
| Glyceryl Stearat, Ceteareth-12, Ceteareth-20, Cetearyl Alcohol, Cetyl Palmitat | | | | | | | 1,50 |
| Glycerinmonostearat (SE) | | 4,00 | | | | | |
| Glyceryl Stearat Citrat | 2,00 | | | | | | |
| Polyglyceryl-3- Methylglucose Distearat | | | | | 2,00 | | |
| Cetearyl Glucosid & Cetearyl Alkohol | | | | | | 2,00 | |
| Triceteareth-4 Phosphat | | | 1,20 | | | | |
| Trilaureth-4 Phosphat | | | | 2,00 | | | |
| Ceteareth-6 | | | 0,50 | 0,50 | | | |
| Sorbitan Stearat | | | 0,75 | 1,00 | 1,00 | | |
| Cetyl Phosphat | | | | | 2,00 | | |
| Stearyl Alkohol | | 2,50 | 3,00 | | | | 1,50 |
| Cetyl Alkohol | 2,50 | 1,00 | | | 0,50 | 2,00 | 2,00 |
| 2-Phenylethyl Benzoat | 1,50 | | | 0,50 | | 4,00 | 10,00 |
| WASorb® K2A | 1,00 | | | 4,00 | | 5,00 | |
| Uvinul® A Plus | 3,00 | 2,50 | 0,50 | 0,25 | 1,00 | 0,50 | |
| Butylmethoxydibenoylmethan | | | | | | | 4,50 |
| Bis-Ethylhexyloxyphenol Methoxyphenyl Triazin | | 2,00 | | | | 1,00 | 0,50 |
| Dinatrium Phenyl Dibenzimidazol Tetrasulfonat | | | | 1,00 | | 2,00 | |
| Phenylbenzmidazol Sulfonsäure | | | | | 3,00 | | |
| Ethylhexyl Triazon | 2,00 | | | 2,00 | | 2,00 | |
| Diethylhexyl Butamido Triazon | | 2,00 | | | | | |
| Ethylhexyl Methoxycinnamat | | 3,50 | | 10,00 | | | |
| Octocrylen | | | | 5,00 | 9,00 | 7,50 | 2,50 |
| Methylen Bis-Benztriazolyl Tetramethylbutylphenol | | | 2,00 | | 3,00 | | |
| Ethylhexylsalicylat | | | | | | | 5,00 |
| Drometrizol Trisiloxan | | | 0,50 | | | 1,00 | |
| Terephtalidene Dicampher Sulfonsäure | | 3,00 | | | | | |
| Dimethylcodiethylbenzalmalona t | | | 5,00 | | | | |
| Titandioxid MT-100Z | 1,00 | | | 3,00 | 1,00 | | |
| Zinkoxid Z-Cote HP1 | | | | | | 3,00 | |
| Corapan TQ® | | | | | | | 6,00 |
| C₁₂₋₁₅ Alkyl Benzoat | | 2,50 | | | | 7,00 | 5,00 |
| Dicaprylylether | | | 3,50 | | 2,00 | | |
| Butylenglykol Dicaprylat/ Dicaprat | 5,00 | | | 5,00 | 3,00 | | |
| Cetearyl Isononanoat | | 4,00 | | | | 2,00 | 2,00 |
| Dimethicon | | 0,50 | 1,00 | | 2,00 | | |
| Cyclomethicon | 2,00 | | | 4,50 | | | 0,50 |
| Dimethicon / Vinyl Dimethicon Crosspolymer | | 4,00 | | | | | 0,50 |
| PVP Hexadecen Copolymer | 0,50 | | | 0,50 | 1,00 | | 1,00 |
| Glycerin | 3,00 | 7,50 | | 7,50 | 5,00 | | 20,00 |
| Xanthangummi | 0,15 | | 0,05 | | | | 0,30 |
| Butylenglycol | 7,00 | 5,00 | | | | 7,00 | |
| Süßholzextrakt | | | | | | 0,5 | |
| Licochalcon | | | 0,05 | | | | |
| Curcumin | 0,05 | | | | | | |
| beta-Carotin | | | | 0,1 | | | |
| Ceramid III | | | | | 0,3 | | |
| Isoserinol | | 1,0 | | | | | |
| Vitamin E Acetat | 0,50 | | 0,25 | 0,50 | 0,75 | | 1,00 |
| Dioic Acid | 0,25 | | | 0,20 | | 0,25 | |
| Fucogel® 1000 | | | 1,50 | | | 5,00 | |
| DMDM Hydantoin | | 0,60 | 0,40 | 0,20 | | | |
| Methylparaben | 0,15 | | 0,25 | | 0,50 | | |
| Phenoxyethanol | 1,00 | 0,40 | | 0,40 | 0,50 | | 0,60 |
| EDTA | | 0,20 | 0,35 | 0,50 | 0,02 | | 0,03 |
| Alkohol | | 2,00 | 1,50 | | 3,00 | 5,00 | 1,00 |
| Insekt Repellent 3535 | | | 5,00 | | | | |
| Meersalz | 0,1 | | | | | | |
| Parfüm | 0,20 | 0,20 | | | | 0,30 | 0,40 |
| Wasser | ad 100 | ad 100 | ad 100 | ad 100 | ad 100 | ad 100 | ad 100 |
| Neutralisationsmittel (NaOH, KOH) | qs | qs | qs | qs | qs | qs | qs |
| pH-Wert | 4,5-6,0 | 4,5-7,0 | 5,5-7,5 | 5,0-7,0 | 5,5-7,5 | 4,0-7,0 | 4,0-7,5 |

## Patentansprüche

1. Kosmetische ÖI-in-Wasser-Emulsion enthaltend
a) 2-Phenylethylbenzoat,
b) ein oder mehrere UV-Filtersubstanzen aus der Gruppe der Triazinderivate,
c) einen O/W-Emulgator oder eine O/W-Emulgatorkombination gewählt aus den folgenden O/W-Emulgatoren:
i) Glycerylstearatcitrat,
ii) Cetearylalkohol + PEG-40 hydriertes Rizinusöl + Natriumcetearylsulfat+ Glycerylstearat,
iii) Polyglyceryl-3-methylglucosedistearat,
iv) Stearinsäure,
v) PEG-40 Stearat
vi) PEG-100 Stearat
vii) Kaliumcetylphosphat.

2. Emulsion nach Anspruch 1, **dadurch gekennzeichnet, dass** als O/W-Emulgator Glycerylstearatcitrat, Cetearylalkohol + PEG-40 hydriertes Rizinusöl + Natriumcetearylsulfat + Glycerylstearat und/oder Polyglyceryl-3-methylglucosedistearat eingesetzt wird.

3. Emulsion nach einem der vorhergehenden Ansprüche, **dadurch gekennzeichnet, dass** sie einen oder mehrere UV-Filtersubstanzen aus der Gruppe der Triazinderivate gewählt aus der Gruppe der Verbindungen 2,4-Bis-{[4-(2-ethyl-hexyloxy)-2-hydroxy]-phenyl}-6-(4-methoxyphenyl)-1,3,5-triazin, Dioctylbutylamidotriazon, 2,4-bis-[5-1 (dimethylpropyl)benzoxazol-2-yl-(4-phenyl)-imino]-6-(2-ethylhexyl)-imino-1,3,5-triazin, 4,4',4"-(1,3,5-Triazin-2,4,6-triyltriimino)-tris-benzoësäure-tris(2-ethylhexylester), enthält.

4. Emulsion nach einem der vorhergehenden Ansprüche, **dadurch gekennzeichnet, dass** sie 2-Phenylethylbenzoat in einer Konzentration von 2 bis 10 Gewichts-%, bezogen auf das Gesamtgewicht der Zubereitung, enthält.

5. Emulsion nach einem der vorhergehenden Ansprüche, **dadurch gekennzeichnet, dass** sie UV-Filtersubstanzen aus der Gruppe der Triazinderivate in einer Konzentration von 0,1 bis 10 bis Gewichts-%, bezogen auf das Gesamtgewicht der Zubereitung, enthält.

6. Emulsion nach einem der vorhergehenden Ansprüche, **dadurch gekennzeichnet, dass** sie O/W-Emulgatoren aus der Gruppe der Emulgatoren i) bis v) in einer Gesamtkonzentration von 0,5 bis 10 Gewichts-%, bezogen auf das Gesamtgewicht der Zubereitung, enthält.

7. Emulsion nach einem der vorhergehenden Ansprüche, **dadurch gekennzeichnet, dass** die Zubereitung weitere UV-Filtersubstanzen enthält.

8. Emulsion nach einem der vorhergehenden Ansprüche, **dadurch gekennzeichnet, dass** sie ein oder mehrere Feuchthaltemittel in einer Gesamtkonzentration von 0,5 bis 10 Gewichts-%, bezogen auf das Gesamtgewicht der Zubereitung, enthält.

## Claims

1. Cosmetic oil-in-water emulsion comprising
a) 2-phenylethyl benzoate,
b) one or more UV filter substances from the group of triazine derivatives;
c) an O/W emulsifier or an O/W emulsifier combination chosen from the following O/W emulsifiers:
i) glyceryl stearate citrate,
ii) cetearyl alcohol + PEG-40 hydrogenated castor oil + sodium cetearyl sulphate + glyceryl stearate,
iii) polyglyceryl-3 methylglucose distearate,
iv) stearic acid,
v) PEG-40 stearate
vi) PEG-100 stearate
vii) potassium cetyl phosphate.

2. Emulsion according to Claim 1, **characterized in that** the O/W emulsifier used is glyceryl stearate citrate, cetearyl alcohol + PEG-40 hydrogenated castor oil + sodium cetearyl sulphate + glyceryl stearate and/or polyglyceryl-3 methylglucose distearate.

3. Emulsion according to one of the preceding claims, **characterized in that** it comprises one or more UV filter substances from the group of triazine derivatives selected from the group of the compounds 2,4-bis{[4-(2-ethylhexyloxy)-2-hydroxy]phenyl}-6-(4-methoxyphenyl)-1,3,5-triazine, dioctylbutylamidotriazone, 2,4-bis[5-1 (dimethylpropyl)benzoxazol-2-yl(4-phenyl)imino]-6-(2-ethylhexyl)imino-1,3,5-triazine, tris(2-ethylhexyl) 4,4',4"-(1,3,5-triazine-2,4,6-triyltriimino)trisbenzoate.

4. Emulsion according to one of the preceding claims, **characterized in that** it comprises 2-phenylethyl benzoate in a concentration of from 2 to 10% by weight, based on the total weight of the preparation.

5. Emulsion according to one of the preceding claims, **characterized in that** it comprises UV filter substances from the group of the triazine derivatives in a concentration of from 0.1 to 10% by weight, based on the total weight of the preparation.

6. Emulsion according to one of the preceding claims, **characterized in that** it comprises O/W emulsifiers from the group of emulsifiers i) to v) in a total concentration of from 0.5 to 10% by weight, based on the total weight of the preparation.

7. Emulsion according to one of the preceding claims, **characterized in that** the preparation comprises further UV filter substances.

8. Emulsion according to one of the preceding claims, **characterized in that** it comprises one or more humectants in a total concentration of from 0.5 to 10% by weight, based on the total weight of the preparation.

## Revendications

1. Emulsion huile-dans-eau cosmétique contenant
a) du benzoate de 2-phényléthyle,
b) une ou plusieurs substances filtrant les UV, choisies dans le groupe des dérivés de triazine,
c) un émulsifiant H/E ou une association d'émulsifiants H/E, choisis parmi les émulsifiants H/E suivants :
i) citrate-stéarate de glycéryle,
ii) alcool cétéarylique + PEG-40 huile de ricin hydrogénée + cétéarylsulfate de sodium + stéarate de glycéryle,
iii) distéarate de polyglycéryl-3-méthylglucose,
iv) acide stéarique,
v) PEG-40 stéarate,
vi) PEG-100 stéarate,
vii) cétylphosphate de potassium.

2. Emulsion selon la revendication 1, **caractérisée en ce qu'**on utilise en tant qu'émulsifiant H/E le citrate-stéarate de glycéryle, l'alcool cétéarylique + PEG-40 huile de ricin hydrogénée + cétéarylsulfate de sodium + stéarate de glycéryle et/ou le distéarate de polyglycéryl-3-méthylglucose.

3. Emulsion selon l'une quelconque des revendications précédentes, **caractérisée en ce qu'**elle contient une ou plusieurs substances filtrant les UV, choisies dans le groupe des dérivés de triazine choisis parmi les composés 2,4-bis{[4-(2-éthyl-hexyloxy)-2- hydroxy]-phényl}-6-(4-méthoxyphényl)-1,3,5-triazine, dioctylbutylamidotriazone, 2,4-bis[5-1(diméthylpropyl)benzoxazol-2-yl-(4-phényl)-imino]-6-(2-éthylhexyl)-imino-1,3,5-triazine, 4,4',4"-(1,3,5-triazine-2,4,6-triyltriimino)-tris-benzoate de tris(2-éthylhexyle).

4. Emulsion selon l'une quelconque des revendications précédentes, **caractérisée en ce qu'**elle contient du benzoate de 2-phényléthyle à une concentration de 2 à 10 % en poids, par rapport au poids total de la préparation.

5. Emulsion selon l'une quelconque des revendications précédentes, **caractérisée en ce qu'**elle contient des substances filtrant les UV, choisies dans le groupe des dérivés de triazine, à une concentration de 0,1 à 10 % en poids, par rapport au poids total de la préparation.

6. Emulsion selon l'une quelconque des revendications précédentes, **caractérisée en ce qu'**elle contient des émulsifiants H/E, choisis dans le groupe des émulsifiants i) à v), à une concentration totale de 0,5 à 10 % en poids, par rapport au poids total de la préparation.

7. Emulsion selon l'une quelconque des revendications précédentes, **caractérisée en ce que** la préparation contient d'autres substances filtrant les UV.

8. Emulsion selon l'une quelconque des revendications précédentes, **caractérisée en ce qu'**elle contient un ou plusieurs agents retenant l'humidité, à une concentration totale de 0,5 à 10 % en poids, par rapport au poids total de la préparation.
